(19) Europäisches Patentamt
European Patent Office
Office européen des brevets

(11) Publication number: **0 259 773 A2**

# (12) EUROPEAN PATENT APPLICATION

(21) Application number: 87112813.8

(22) Date of filing: 02.09.87

(51) Int. Cl.⁴: C12P 21/00 , C07K 13/00 , G01N 33/577 , G01N 33/574

(30) Priority: 10.09.86 JP 211694/86

(43) Date of publication of application: 16.03.88 Bulletin 88/11

(84) Designated Contracting States: AT BE CH DE ES FR GB GR IT LI LU NL SE

(71) Applicant: Asahi Kasei Kogyo Kabushiki Kaisha
2-6, Dojimahama 1-chome Kita-ku
Osaka-shi Osaka 530(JP)

(72) Inventor: Kaieda, Takeji
168-52 Obuchi
Fuji-shi Shizuoka-ken(JP)
Inventor: Morimoto, Takuji
351-1, Samejima
Fuji-shi Shizuoka-ken(JP)
Inventor: Yamawaki, Naokuni
3-7, Fujimidai
Fuji-shi Shizuoka-ken(JP)

(74) Representative: Boeters, Hans Dietrich, Dr. et al
Boeters, Bauer & Partner
Thomas-Wimmer-Ring 14
D-8000 München 22(DE)

## (54) A novel antibody specific for a cancer antigen.

(57) A novel antibody capable of binding to an antigen which is also novel and specifically present in the serum of a patient suffering from a cancer is disclosed. Using the antibody of the present invention, the antigen can be detected easily and effectively. Therefore, the antibody of the present invention is useful for the diagnosis of a cancer using as a criterion the presence of the antigen.

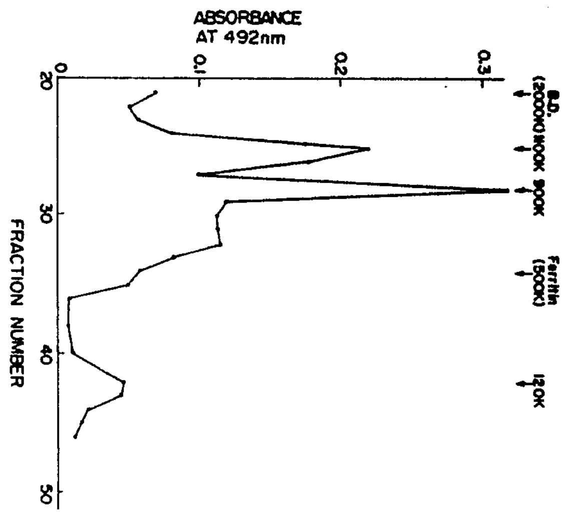


FIG. 2

EP 0 259 773 A2

Xerox Copy Centre

# A NOVEL ANTIBODY SPECIFIC FOR A CANCER ANTIGEN

## Background of The Invention

### Field of The Invention

This invention relates to a novel antibody specific for a cancer antigen. More particularly, the present invention is concerned with a novel antibody capable of binding to an antigen which is also novel and specifically present in the sera of patients suffering from cancers. The antibody can be advantageously utilized for clinical examination to determine whether a patient suffers from a cancer, using as a criterion the presence of the antigen in the body of the patient. The present invention also relates to a novel antigen as mentioned above.

### Discussion of Related Art

Conventionally, there are known several tumor markers which are specifically present in the serum of a patient suffering from a cancer. As such tumor markers, there may be mentioned, for example, α-fetoprotein (hereinafter often referred to as "AFP") and carcinoembryonic antigen (hereinafter often referred to as "CEA") which were found in 1960's. As mentioned above, the tumor markers are specific for the serum of a patient suffering from a cancer. Therefore, the tumor markers are a useful index for the diagnosis of cancer. In fact, an assay kit for detecting the tumor markers is now produced and sold on a market, and used for the estimation of the result of treatment for a cancer, the early detection of relapse of a cancer, the determination of the portion at which a cancer occurs, etc. Further, in the 1980's, another tumor marker CA19-9 has been found which is a useful index for the diagnosis of cancer of the pancreas [B.F. Atkinson et al, Cancer Res., 42, 4820 (1982)].

However, when the diagnosis of various cancers is conducted using as a criterion the presence of the above-mentioned conventional tumor markers in a specimen such as serum derived from a human body, it is highly possible that errors in diagnosis occur. The reason for this is as follows. For example, the above-mentioned CEA has been frequently utilized as an index for a clinical examination to determine whether a patient suffers from colon carcinoma or gastric cancer. However, the positive ratio (the ratio of the number of cancer patients, whose sera are found to contain the tumor marker, to the number of cancer patients whose sera are subjected to examination to determine whether the sera contain the tumor marker) is as low as about 50 % in the case of a colon carcinoma and about 30 % in the case of a gastric cancer. On the other hand, the AFP has frequently been utilized as an index for the clinical examination to determine whether a patient suffers from a hepatoma. However, the AFP-positive sera are often found in patients suffering from hepatic diseases other than a hepatoma. That is, the specificity of AFP to a hepatoma is low and, hence, the positive ratio of a hepatoma obtained using, as a criterion, the presence of AFP in serum is also low as in the case of the above-mentioned CEA. Under these circumstances, it has been earnestly desired in the art to obtain a tumor marker which is present specifically in the serum of a patient suffering from a certain kind of cancer so that a high positive ratio can be achieved, and to develop an assay kit for detecting such a tumor marker to diagnose a certain kind of cancer with high specificity and with high probability. Further, it has also been desired in the art to obtain a tumor marker which is present in the sera of various patients suffering from various kinds of cancers and to develop an assay kit for detecting such a tumor marker to diagnose various kinds of cancers with high probability. However, heretofore, such a convenient tumor marker has not yet been obtained and, hence, there has not yet been obtained any assay kit for detecting such a tumor marker which is useful for the diagnosis of various kinds of cancers with high specificity and high probability.

## Summary of The Invention

The present inventors have made extensive and intensive studies to solve the above-mentioned problems. To this end, the present inventors conducted extensive and intensive research for a novel convenient tumor marker by a method in which various hybridomas capable of producing a monoclonal antibody against ConA-binding glycoproteins obtained from the supernatant of the culture of the human tumor cell line NBT-2 are prepared, followed by screening. Particularly, the ConA-binding glycoproteins are obtained from the supernatant of the culture of the human tumor cell line NBT-2 and injected into a BALB/c mouse several times to immunize the mouse. Then, spleen cells are obtained from the immunized mouse. The thus obtained spleen cells are fused with cells of a mouse myeloma strain P3U1 to obtain various hybridomas each producing a monoclonal antibody, followed by screening. As a result, it has been found that some of the hybridomas produce a monoclonal antibody which is reactive with the sera of patients suffering from a cancer but not reactive with a normal human serum. Further, the present inventors have made studies on a substance in the sera of cancer patients, which is capable of binding to the above-mentioned monoclonal antibodies. As a result, it has been found that as a novel tumor marker, there are at least four antigens which are present in the sera of patients suffering from a cancer, but scarcely present in normal human serum. Also, it has been found that antigens are present in the supernatants of cultures of various human tumor cell lines. Further, it has also been found that using the above-mentioned monoclonal antibody, the diagnosis of a cancer can be effectively conducted with high probability. The present invention has been made based on such novel findings.

Therefore, it is an object of the present invention to provide an antibody against a novel antigen of the kind as mentioned above.

It is another object of the present invention to provide a novel antigen of the kind as mentioned above.

It is a further object of the present invention to provide a method for detecting the antigen of the kind as mentioned above for the diagnosis of a cancer.

The foregoing and other objects, features and advantages of the present invention will be apparent from the following detailed description taken in connection with the accompanying drawings.

## Brief Description of The Drawings

In the drawings:

Fig. 1 is a graph showing the relationship between the concentration of an antigen of the present invention and the absorbance at 492 nm which is obtained by immunoassay as described later;

Fig. 2 is a graph showing the result of the determination of the molecular weight of an antigen of the present invention by gel filtration chro matography, which antigen is obtained from the supernatant of the culture of a human tumor cell line NBT-2;

Fig. 3 is a graph showing the result of the determination of the molecular weight of an antigen of the present invention by gel filtration chromatography, which antigen is obtained from the serum of a patient suffering from a lung cancer;

Fig. 4 is a graph showing the result of the determination of the molecular weight of an antigen of the present invention by gel filtration chromatography, which antigen is obtained from the serum of a patient suffering from a breast carcinoma;

Fig. 5 is a graph showing the result of the chromatofocusing of an antigen of the present invention having a molecular weight of about 900,000 which is obtained from the serum of a patient suffering from a breast carcinoma;

Figs. 6-(A), 6-(B) and 6-(C) are graphs showing the binding activity of an antigen of the present invention to lectins ConA, PNA and PWA, respectively, which antigen is obtained from the supernatant of the culture of a human tumor cell line NBT-2;

Fig. 7 is a graph showing the results of the determination of the antigen concentration in the sera of patients each suffering from one of various cancers; and

Figs. 8-(A), (B) and (C) are graphs showing the results of an enzyme immunoassay in which various fractions containing an antigen of the present invention which are obtained, using a lectin, from the supernatant of a culture of a human tumor cell line NBT-2 are subjected to assay.

## Detailed Description of The Invention

Essentially, according to the present invention, there is provided an antibody which is capable of binding to at least one antigen selected from the group consisting of:

(A) a glycoprotein having a molecular weight of about 1,100,000 ± 100,000 and having an isoelectric point of about 4.0 ± 0.2,

(B) a glycoprotein having a molecular weight of about 900,000 ± 100,000 and having an isoelectric point of about 4.2 ± 0.2,

(C) a glycoprotein having a molecular weight of about 700,000 ± 100,000 and having an isoelectric point of about 4.8 ± 0.2, and

(D) a glycoprotein having a molecular weight of about 120,000 ± 20,000 and having an isoelectric point of about 6.5 ± 0.5;

and wherein at least one of said antigen is present in the serum of a patient suffering from a hepatoma, a mastocarcinoma, a gastric cancer or a lung cancer, and wherein at least one of said antigen is capable of binding to at least one monoclonal antibody selected from the group consisting of:

a monoclonal antibody produced by the hybridoma N2421 deposited at the European Collection of Animal Cell Cultures under the accession number 87050805;

a monoclonal antibody produced by the hybridoma N7072 deposited at the European Collection of Animal Cell cultures under the accession number 87012801; and

a monoclonal antibody produced by the hybridoma N9261 deposited at the European Collection of Animal Cell Cultures under the accession number 87012802.

Also, according to the present invention, there is provided an antigen which is a glycoprotein present in the serum of a patient suffering from a hepatoma, a breast carcinoma, a gastric cancer or a lung cancer, and is capable of binding to at least one monoclonal antibody selected from the group consisting of:

a monoclonal antibody produced by the hybridoma N2421 deposited at the European Collection of Animal Cell Cultures under the accession number 87050805;

a monoclonal antibody produced by the hybridoma N7072 deposited at the European Collection of Animal Cell Cultures under the accession number 87012801; and

a monoclonal antibody produced by the hybridoma N9261 deposited at the European Collection of Animal Cell Cultures under the accession number 87012802.

Further, according to the present invention, there is provided a method for detecting an antigen of the kind as mentioned above in a specimen derived from a human body, which comprises subjecting the specimen to immunoassay using an antibody of the kind as mentioned above.

Further, according to the present invention, there is provided a method for producing an antigen of the kind as mentioned above, which comprises determining the presence of an antigen of the kind as mentioned above in a serum of a patient suffering from a cancer or a supernatant of a culture of a human tumor cell line, and subjecting the serum or supernatant to purification using a lectin.

The antibody of the present invention is capable of binding to at least one antigen selected from the group consisting of:

(A) a glycoprotein having a molecular weight of about 1,100,000 ± 100,000 and having an isoelectric point of about 4.0 ± 0.2,

(B) a glycoprotein having a molecular weight of about 900,000 ± 100,000 and having an isoelectric point of about 4.2 ± 0.2,

(C) a glycoprotein having a molecular weight of about 700,000 ± 100,000 and having an isoelectric point of about 4.8 ± 0.2, and

(D) a glycoprotein having a molecular weight of about 120,000 ± 20,000 and having an isoelectric point of about 6.5 ± 0.5.

The above-mentioned antigen is specifically present in the serum of a patient suffering from a cancer. At least, the antigen is present in the serum of a patient suffering from a hepatoma, a breast carcinoma, a gastric cancer or a lung cancer. However, the antigen is scarcely present in normal human serum or, if any, the amount of the antigen in normal human serum is extremely small. The antigen is also present in the supernatant of a culture of a human tumor cell line. Particularly, the antigen is present in the supernatant of a culture of a human tumor cell line PC-1, PC-13, PC-14 or NBT-2 in relatively high amount. The cell lines PC-1, PC-13 and PC-14 are derived from human lung cancers, and the cell line NBT-2 is derived from a cancer of the human urinary bladder. However, the antigen is scarcely present in the supernatants of cultures of human tumor cell lines KATO-III and MKN-45 derived from gastric cancers.

The above-mentioned antigens are capable of binding to at least one of the following monoclonal antibodies:

(a) a monoclonal antibody produced by the hybridoma N2421 deposited at the European Collection of Animal Cell Cultures under the accession number 87050805;

(b) a monoclonal antibody produced by the hybridoma N7072 deposited at the European Collection of Animal Cell Cultures under the accession number 87012801; and

(c) a monoclonal antibody produced by the hybridoma N9261 deposited at the European Collection of Animal Cell Cultures under the accession number 87012802.

Of the above-mentioned monoclonal antibodies, the monoclonal antibodies produced by the hybridomas N2421 and N7072 are capable of binding to all of the above-mentioned 4 antigens. This means that the 4 antigens have a common epitope, which the monoclonal antibodies produced by the hybridomas N2421 and N7072, can recognize and bind to. On the other hand, the monoclonal antibody produced by the hybridoma N9261 is capable of strongly binding to the above-mentioned antigens (A), (C) and (D), but the binding ability of the monoclonal antibody produced by the hybridoma N9261 to the above-mentioned antigen (B) is weak. The above-mentioned monoclonal antibodies have been obtained in researching a novel tumor marker as mentioned above using a customary cell fusion technique. That is, using as an antigen solution the supernatant of a culture of the human tumor cell line NBT-2, the immunization of a BALB/c mouse is conducted. Then, from the immunized mouse, a spleen is taken out and the cells of the spleen are fused with cells of a mouse myeloma strain P3U1 (which is publicly available from the Immuno Biological Laboratories Co., Ltd., Japan) to obtain hybridomas. The hybridomas are subjected to screening to obtain various hybridomas which produce an antibody reactive with the sera of patients suffering from various cancers. Of the hybridomas, those which produce an antibody which is strongly reactive with the sera of the patients suffering from cancers are selected. Thus, there are obtained the above-mentioned hybridomas N2421, N7072 and N9261. Thereafter, a search has been conducted for an antigen present in the serum of a patient suffering from a cancer, which antigen is capable of binding to the antibodies produced by those hybridomas by enzyme immunoassay using those antibodies. As a result, it has been found that there are at least 4 antigens (A), (B), (C) and (D) having the molecular weights and isolelectric points as mentioned above. Those antigens are collectively named "ATM-1". The antibody of the present invention is capable of binding to at least one of the ATM-1 antigens.

The antibody of the present invention may be produced by any customary method using the ATM-1. For example, the antibody may be produced as follows. The ATM-1 is injected into an animal such as a mouse, rabbit, etc. several times at predetermined intervals (generally 1 to 2 weeks) to immunize the animal. From the immunized animal, serum is collected. The collected serum is an antiserum against ATM-1. The antiserum is subjected to purification by any customary method, for example, salting-out using ammonium sulfate, ion exchange chromatography using a column packed with DEAE-Sepharose, etc.

The antibody of the present invention may also be produced from the culture of a hybridoma capable of producing the antibody of the present invention. For example, the antibody of the present invention may be produced as follows. A hybridoma capable of producing the present antibody is transplanted to the abdominal cavity of a BALB/c mouse. 10 to 14 days later, the ascites of the mouse is collected and subjected to purification by any customary method as mentioned before. According to a method of producing an antibody of the present invention in which the hybridoma is used, there is obtained a monoclonal antibody (hereinafter referred to as "anti-ATM-1 monoclonal antibody"). For the commercial production of the present antibody, the method using a hybridoma is advantageous.

The hybridoma capable of producing an anti-ATM-1 monoclonal antibody may be obtained by a customary method, for example, a method of Köhler et al [G. Köhler and C. Milstein: Nature, 256, 495 (1975)]. Particularly, a human tumor cell line such as NBT-2 is cultured, and the culture is subjected to centrifugation to collect a supernatant. From the supernatant, a ConA-binding glycoprotein fraction is obtained by a customary fractionation method using ConA lectin, for example by a chromatography method, a batchwise fractionation method, etc. The glycoprotein fraction is subcutaneously injected to a BALB/c mouse together with the Freund's complete adjuvant. Two weeks later, the glycoprotein fraction is once more injected to the mouse intravenously. Three days later, the spleen of the mouse is excised, and the cells of the spleen are subjected to cell fusion with cells of a mouse myeloma P3U1 in the presence of polyethylene glycol to form hybridomas. The thus formed hybridomas are subjected to screening to select a hybridomas capable of producing an anti-ATM-1 monoclonal antibody as follows. Each of the hybridomas is cultured and the culture is subjected to centrifugation to obtain a supernatant. The supernatant is subjected to enzyme immunoassay (EIA) using a purified ATM-1 to determine whether the supernatant contains an anti-ATM-1 monoclonal antibody. The purified ATM-1 which is used in the EIA may be obtained as explained hereinbelow. The hybridomas, from which the supernatant containing the anti-ATM-1 monoclonal antibody is obtained, are selected. Thus, there are obtained hybridomas capable of producing an anti-ATM-1 monoclonal antibody.

As the hybridomas capable of producing an anti-ATM-1 monoclonal antibody, there may be mentioned, for example, hybridomas N2421, N7072, N9261, N1977, N3216, N1019, N3233 and the like. The hybridomas N2421, N7072 and N9261 are deposited at the European Collection of Animal Cell Cultures under the accession numbers 87050805, 87012801 and 87012802, respectively.

The antigen of the present invention, namely, the ATM-1, is present in the serum of a patient suffering from a hepatoma, a breast carcinoma, gastric cancer or a lung cancer. The present antigen is also present in the supernatant of a culture of a human tumor cell line PC-1, PC-13, PC-14 or NBT-2.

The antigen of the present invention is capable of binding to at least one of the abovementioned anti-ATM-1 monoclonal antibodies.

Further, the present antigen has the following characteristics.

(1) The antigen is capable of binding to at least two lectins, namely, ConA lectin and wheat germ agglutinin (WGA). This fact indicates that the present antigen is a glycoprotein.

(2) The antigenicity of the antigen against the monoclonal antibody produced by the hybridoma N2421 is stable at least at 56 °C for 30 min.

The antigen of the present invention may be produced from either the serum of a patient suffering from a cancer as mentioned above, or the supernatant of a culture of a human tumor cell line as mentioned above. Further, the present antigen may also be produced from the serum of an animal, for example, sheep etc. However, from the standpoint of availability of the raw material, it is preferred to produce the antigen from the supernatant of the culture of a human tumor cell line.

The human tumor cell lines which may be used for the production of the present antigen are, for example, PC-1, PC-13, PC-14 and NBT-2. However, the human tumor cell line is not limited to those cell lines, and needless to say, any cell lines may be used as long as they can produce the present antigen.

The supernatant of a culture of a human tumor cell line is obtained as follows. A human tumor cell line is cultured in a customary nutrient medium, for example, an RPMI-1640 medium containing 10 v/v% fetal calf serum, an MEM medium containing 10 v/v% fetal calf serum and a commercially available synthetic medium containing no serum. The culturing may be conducted at about 37 °C for 3 to 5 days. The resulting culture is subjected to centrifugation to separate into a supernatant and cells, and the supernatant is then collected.

On the other hand, the serum of a patient suffering from a cancer may be obtained as follows. Blood is collected from the patient. The blood is subjected to centrifugation to separate the serum, and the serum is collected.

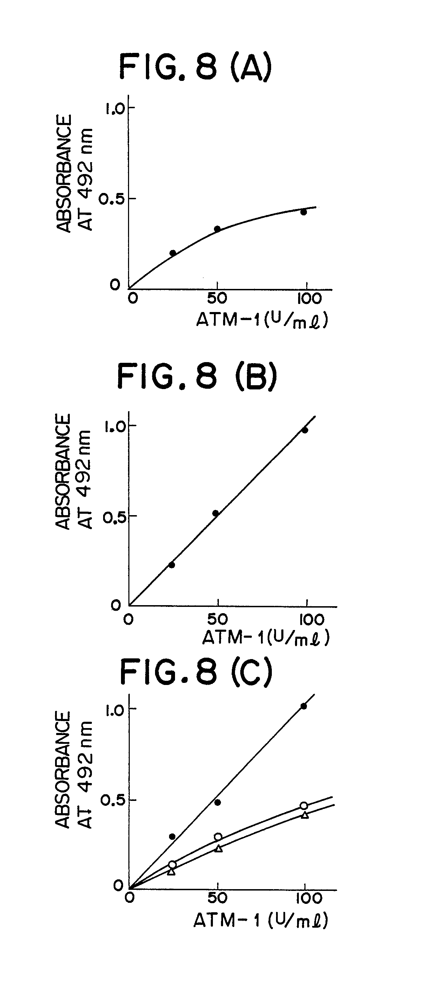

From the thus obtained supernatant or serum, the antigen of the present invention may be produced by subjecting the supernatant or serum to purification by customary methods, for example, gel chromatography, electrophoresis, ion exchange chromatography, adsorption chromatography, affinity chromatography using an anti-ATM-1 monoclonal antibody-linked bead and the like. However, it is advantageous to purify the antigen using a lectin, because, as discussed above, the antigen is a glycoprotein and is capable of specifically binding to a lectin. For example, the serum or supernatant is contacted with a lectin to adsorb the antigen on the lectin and the resulting lectin is washed. Then, the antigen is eluted out from the lectin using, as an eluent, a saccharide-containing solution. The lectins used in the present invention are, for example, ConA, WGA and the like. Generally, the lectin may be used in the form of being linked with a bead such as Sepharose (manufactured and sold by Pharmacia Fine Chemicals AB, Sweden). As the saccharide-containing solution, there may be employed, for example, a saccharide-containing phosphate buffer and the like. As a saccharide, there may be employed, for example, α-methylmannose and the like. In carrying out the purification using a lectin, it should be noted that in the serum and supernatant there are glyco proteins other than the present antigen, and those glycoproteins are also adsorbed on a lectin together with the present antigen. Those glycoproteins often accompany the present antigen as impurities to decrease the sensitivity of EIA for the diagnosis of a cancer in which the present antigen is used as a standard substance. These glycoproteins are hereinafter referred to as "EIA inhibitory substances". See Figs. 8(A) to (C). Therefore, it is necessary to remove these glycoproteins from the present antigen. These glycoproteins are weak in ability to bind to a lectin as compared with the present antigen. Therefore, the removal of such glycoproteins other than the antigen of the present invention may be conducted as follows. After the contact of the serum or supernatant with a lectin, the lectin is washed with a 0.4 M saccharide solution to elute out from the lectin the glycoproteins other than the present antigen. Thereafter, from the resulting lectin, the present antigen is eluted out, using as an eluent, a saccharide solution having a saccharide concentration of 0.4 M or more. In the case where it is intended to obtain the present antigen having extremely high purity, before eluting out using a 0.4 M saccharide solution, the lectin may be washed with a buffer containing no saccharide and/or a saccharide solution having a saccharide concentration of less than 0.4 M.

Incidentally, in the case where the antigen of the present invention is produced from the serum, the serum as such may be subjected to purification. Alternatively, the serum may be diluted about ten times with a phosphate buffered saline (hereinafter referred to as "PBS") before subjecting to purification.

Thus, there is obtained the antigen of the present invention, namely the ATM-1, in substantially pure form. That is, the present antigen thus obtained has a high purity such that when the present antigen is used as a standard antigen in the immunoassay as described later, there is obtained a linear relationship between the amount of the antigen and the absorbance obtained by the immunoassay. Therefore, the present antigen is useful as a standard substance for preparing a standard curve used in the immunoassay for detecting the antigen in a specimen derived from a patient. The immunoassay for detecting the antigen will be explained hereinbelow.

As shown in Figs. 2 to 4, the antigen of the present invention includes glycoproteins respectively having molecular weight of about 120,000 and about 500,000 to about 1,500,000. The glycoproteins having molecular weights of about 500,000 to about 1,500,000 have isoelectric points of about 3.8 to 5.0. As is apparent from the peaks of absorbance shown in Figs. 2 to 4, the antigen of the present invention includes at least four glycoproteins (A) to (D) as mentioned before. The glycoproteins (A), (B), (C) and (D) have molecular weights of about 1,100,000 ± 100,000, about 900,000 ± 100,000, about 700,000 ± 100,000 and about 120,000 ± 20,000, respectively. Further, the glycoproteins (A), (B), (C) and (D) have isoelectric points of about 4.0 ± 0.2, about 4.2 ± 0.2, about 4.8 ± 0.2 and 6.5 ± 0.5, respectively. The molecular weight and isoelectric point of the antigen of the present invention are determined by a gel filtration chromatography method and a chromatofocusing method, respectively. The gel filtration chromatography method and the chromatofocusing method used herein will be explained hereinbelow.

(1) Gel filtration chromatography method:

0.5 ml of a sample solution containing the antigen of the present invention is applied to a column (1.5 cm $\phi \times$ 90 cm) packed with Sepharose CL6B(manufactured and sold by Pharmacia Fine Chemicals AB, Sweden). Then, the elution is conducted using as an eluent 10 mM phosphate buffer (pH7.4) containing 0.85 w/v% NaCl (phosphate buffered saline)(hereinafter referred to as "PBS"), and 2.5 ml fractions are collected. Each fraction is condensed to a volume of one fifth using Molcut SJTK 01324 (an ultrafiltration unit manufactured and sold by Nihon Millipore Ltd., Japan). The condensed fraction is subjected to enzyme immunoassay as described later in Example 5 to determine the amount of the antigen of the present invention in each fraction. Thus, there is obtained a chromatogram. Incidentally, as the standard molecular weight markers, blue dextran (B.D., molecular weight: 2000k), ferritin (molecular weight: 500k) and cytochrome c (molecular weight: 12.5k) are applied to the above-mentioned column before the sample solution is applied to the column to obtain a molecular calibration pattern. By the comparison of the chromatogram with the molecular calibration pattern, there is obtained a molecular weight of the antigen of the present invention.

(2) Chromatofocusing method:

The chromatofocusing is conducted using Polybuffer 74 and PBE94 (each manufactured and sold by Pharmacia Fine Chemicals AB, Sweden) as follows. A column (1 cm $\phi \times$ 20 cm) packed with PBE94 is equilibrated with an initiation buffer [25 mM imidazole-HCl (pH7.4)]. The solvent of a sample solution containing the present antigen is replaced by a Polybuffer 74-HCl (pH7.0) using Molcut SJTK 01324 (manufactured and sold by Nihon Millipore Ltd., Japan) and the resulting sample solution is applied to the above-mentioned column. The elution is conducted using as an eluent a Polybuffer 74-HCl (pH7.0), and 1.5 ml fractions are collected. Each fraction is subjected to measurement of the pH value. Then, each fraction is neutralized and diluted 25-fold with PBS. Then, the resulting solution is condensed to 0.3 ml using the abovementioned Molcut. The thus obtained fraction is subjected to enzyme immunoassay as described in Example 5 (which will be given later) to determine the amount of the antigen in the sample. The pH of the fraction which contains the antigen in the highest amount represents the isolectric point (pI) of the antigen.

As mentioned before, the antigen of the present invention is present in the supernatant of a culture of a human tumor cell line. The antigen is also found to be present in the cell extract of at least human tumor cell lines NBT-2 and PC-14. That is, the cells of each of the human tumor cell lines NBT-2 and PC-14 are lysed using a nonionic surfactant Triton X-100 to obtain a cell extract. The cell extract is subjected to SDS-polyacrylamide gel electrophoresis. Then, from the resulting gel, the electrophoresed substances are transferred to a nitrocellulose membrane. Then, the antigen on the nitrocellulose membrane, which is reactive to the monoclonal antibody of the present invention, is detected using a monoclonal antibody produced by the above-mentioned hybridoma N2421 and labeled with peroxidase. As a result, it is found that in the cell extract, there exists at least one antigen of the present invention reactive to the monoclonal antibody. This antigen has a molecular weight of about 20,000 to 50,000 and, therefore, is of a kind different from the antigens (A) to (D) as mentioned before.

As mentioned before, the antigen, ATM-1, of the present invention is specifically present in the serum of a patient suffering from a cancer, but is scarcely present in normal human serum and the serum of a patient suffering from a disease other than a cancer as shown in Fig. 7. Therefore, for the diagnosis of a cancer, it is useful to determine whether or not the antigen ATM-1 is present in the serum of a patient. According to the present invention, such determination can be conducted by a method for detecting the antigen in a specimen derived from a human body which comprises subjecting the specimen to immunoassay using the antibody of the present invention.

As the specimen derived from a human body, a serum is generally employed. In addition, tissues and body fluids such as sputum, urine and the like may also be used as specimens.

As the antibody used in the immunoassay, there are employed any one of the above-mentioned antibodies of the present invention. Further, there may also be employed an antiserum obtained from an animal immunized with the ATM-1 and any antibody fraction which is obtained by subjecting the antiserum to purification by the above mentioned methods. However, from the standpoint of specificity to a cancer and reproducibility of the detection, it is preferred to use a monoclonal antibody. In the case where a monoclonal antibody is used, the immunoassay may be conducted by the use of one kind of monoclonal antibody. However, from the standpoint of sensitivity and accuracy, it is preferred to use 2 kinds of monoclonal antibodies. The immunoassay using the antibody of the present invention may be conducted by various customary methods according to the description of a textbook "Koso Men-eki Sokuteiho (Enzyme Immunoassay)," edited by E. Ishikawa, published by Igaku Shoin, Tokyo (1982). Hereinbelow, a generally employed sandwich method in which 2 kinds of monoclonal antibodies are used will be explained. One of the monoclonal antibodies is fixed as a primary antibody to a plate or bead used for immunoassay. To the resulting plate or bead is added a specimen derived from a human body, followed by incubation at 4 to 37 °C for a predetermined period. The plate or bead is washed. Then, to the plate or bead is added, as a secondary antibody, another kind of the antibody which is labeled with an enzyme such as peroxidase, a radioisotope such as $^{125}I$, biotin or the like. As the secondary antibody, the antibody of the present invention may be used. Alternatively, the F(ab')2 or F(ab') portion of the antibody obtained by removing the Fc portion of the antibody by digestion with pepsin may also be used. The labeling of the antibody or its $F(ab')_2$ or F-(ab') portion may be conducted by a customary method as described, for example, in a textbook "Zoku-Seikagaku Jikken Koza 5, Men-eki Seikagaku Kenkyuho (Lectures on Experimental Biochemistry, Second series, Vol. 5, Immunological and biochemical Assays)", edited by the Japanese Society of Biochemistry, published by Tokyo Kagaku Dojin, Japan (1986), pp.102-111]. In the case where only one kind of monoclonal antibody is used for the immunoassay, the amount of the monoclonal antibody may generally be about 1 µg/ml to 100 µg/ml. On the other hand, in the case where 2 kinds of monoclonal antibodies are used for the immunoassay, the amount of the primary antibody may generally be about 1 µg/ml to 100 µg/ml and the amount of the secondary antibody may generally be about 0.1 µg/ml to 100 µg/ml.

The antigen (ATM-1) of the present invention is not reactive to the antibodies which are used in the conventional assay kits for detecting the following known tumor markers.

| Tumor marker | Manufacturer of an assay kit for detecting the tumor marker |
|---|---|
| CEA | Dinabbot Co., Japan |
| AFP | " |
| CA19-9 | Fujirebio Inc., Japan |
| CA125 | Centocor Co., U.S.A. |
| CA15-3 | " |

Further, the tumor marker CA15-3, one of the conventional tumor markers, is present in a milk fatty globule of a patient suffering from a breast carcinoma. However, the antigen of the present invention is not found in the milk fatty globule of a patient suffering from a breast carcinoma. Moreover, it is known that there are tumor-associated antigens on the surface of a tumor cell membrane. The presence of the tumor-associated antigens is determined by reacting a fluorescein isothiocyanate (FITC)-bonded anti-tumor-associated antigen monoclonal antibody with a tumor cell membrane and detecting the fluorescence derived from FITC-bonded antibody which binds to the tumor-associated antigens on the surface of the tumor cell membrane. However, an FITC-bonded monoclonal antibody obtained from a hybridoma N2421 (one of anti-ATM-1 monoclonal antibodies) is not reacted with a tumor cell membrane, that is, the antigen of the present invention is not present on the surface of a tumor cell membrane as is different from the known tumor-associated antigens. These facts indicate that the antigen of the present invention is novel and completely different from the conventionally known tumor markers and tumor-associated antigens as mentioned above.

The specificity of the antigen of the present invention to the specimen derived from a human body of a patient suffering from a cancer is extremely high as compared with those of the conventional markers. The amount of the antigen(ATM-1) of the present invention in the serum of a patient suffering from a cancer is relatively large and sometimes as large as about 200 U/ml. By contrast, in normal human serum, the ATM-1 is scarcely present and, if any, 5 U/ml or less. The unit "U" used herein means the amount of the ATM-1 one-thousandth that of the ATM-1 prepared from 1 ℓ of the supernatant of the culture of a human tumor cell line NBT-2 in Example 1 which will be discussed hereinbelow. If the serum containing the present antigen in an amount of more than 5 U/ml is defined as being ATM-1 positive, the positive ratio of the present antigen (the ratio of the number of the cancer patients, whose sera are found to contain the ATM-1, to the number of the cancer patients whose sera are subjected to examination to determine whether the sera contain the ATM-1) is extremely high as compared with those of the conventional tumor markers. For example, the positive ratio is as high as 90 % or more in the case of a hepatoma, 70 % or more in the case of a breast carcinoma, 60 % or more in the case of a gastric cancer and 50 % or more in the case of a lung cancer. Therefore, for the diagnosis of a cancer it is advantageous to detect the present antigen as compared with the detection of the conventional tumor markers.

The antibody of the present invention is advantageous to specifically detect such an antigen ATM-1 in the specimen derived from a human body. According to the method of the present invention for detecting the antigen ATM-1 using the antibody of the present invention, the diagnosis of a cancer can be carried out with accuracy.

Hereinafter, the present invention will now be described in detail with reference to the following Examples, which should not be construed to be limiting the scope of the present invention.

In the following Examples, the human tumor cell lines used are all publicly available from the Immuno Biological Laboratories, Co., Ltd.; 1091-1, Aza-Tabata, Naka, Fujioka-shi, Gumma-ken, Japan.

The following hybridomas of the present invention obtained in the Examples have been deposited at the European Collection of Animal Cell Cultures, Public Health Laboratory Service Center for Applied Microbiology and Research (Porton Down, Salisbury Wiltshire, SP40JG, U.K.)

| Hybridoma | Deposit No. |
|---|---|
| Hybridoma N2421 | 87050805 |
| Hybridoma N7072 | 87012801 |
| Hybridoma N9261 | 87012802 |

Example 1

Production of an ATM-1 from a supernatant of a culture of a human tumor cell line NBT-2:

Run 1 (Preparation of purified ATM-1)

Cells of a human bladder tumor cell line NBT-2 were suspended in an RPMI 1640 medium - (manufactured and sold by Nissui Pharmaceutical Co., Ltd., Japan) containing 10 v/v% fetal calf serum (hereinafter referred to as "FCS") in a culture bottle (catalogue No. Nunc 56502, manufactured and sold by Nunc Co., Ltd., Denmark), so that the cell concentration of the resutling suspension became 1 $\times$ $10^5$ cells/ml. The suspension was incubated at 37 °C for 5 days under the atomsphere of 5 v/v% $CO_2$. Then, the resulting culture was centrifuged at 800 r.p.m. for 10 min. to separate the culture into a supernatant and precipitates. To 5 ℓ of the supernatant was added 50 ml of ConA-Sepharose beads (manufactured and sold by Pharmacia Fine Chemicals AB, Sweden), and the mixture was shaken at 4 °C for 15 hours so that glycoproteins present in the supernatant were adsorbed on the ConA-Sepharose beads. Then, the mixture comprising the supernatant and the ConA-Sepharose beads on which the glycoproteins were adsorbed was subjected to suction-filtration using a glass filter (Hario G3, manufactured and sold by Shibata Hario Glass Co., Ltd., Japan) to obtain, on the glass filter, the ConA-Sepharose beads on which the glycoproteins were adsorbed. Through the glass filter, 3 ℓ of 10 mM phosphate buffer, pH 7.4 (hereinafter referred to as"PB") was flowed to wash away impurities on ConA-Sepharose beads. The washed ConA-Sepharose beads were collected and suspended in 500 ml of a PB solution containing 0.2 M α-methylmannose (manufactured and sold by Sigma Chemical Company, U.S.A.), followed by shaking at room temperature for 2 hours. The resulting suspension was subjected to suction-filtration using the same glass filter as mentioned above, thereby to separate the ConA-Sepharose beads from a filtrate (0.2 M α-methylmannose-eluted eluate). Then, the ConA-Sepharose beads on the glass filter were collected and suspended in 500 ml of a PB solution containing 0.4 M α-methylmannose, and the thus obtained suspension was shaken at room temperature for 2 hours, followed by suction-filtration using the same glass filter as mentioned above, thereby to separate the ConA-Sepharose beads from a filtrate (0.4 M α-methylmannose-eluted eluate). Then, the resulting ConA-Sepharose beads were collected and suspended in 500 ml of a PB solution containing 0.5 M α-methylmannose, and the thus obtained suspension was shaken at room temperature for 2 hours so that an ATM-1 which was adsorbed on the ConA-Sepharose beads was eluted out therefrom. Then, the suspension was subjected to suction-filtration using the same glass filter as mentioned above, thereby to separate a filtrate (0.5 M α-methylmannose-eluted eluate containing the ATM-1) from the ConA-Sepharose beads, which were returned for re-use. The thus obtained filtrate was subjected to ultrafiltration using a ultrafilter (Amicon PM-10, manufactured and sold by Amicon Co., Ltd., U.S.A.) to concentrate the filtrate. The concentrated filtrate was dialysed against PB to remove α-methylmannose therefrom. Thus, there was obtained 5 ml of an α-methylmannose-free PB solution containing the ATM-1. The protein concentration of the thus obtained solution was 1.2 mg/ml as determined based on an absorbance at 280 nm. The amount of the purified ATM-1 per ml of the thus obtained solution was defined as 1000 U. The ATM-1 thus purified was designated NBT-ATM-1. In order to determine whether the NBT-ATM-1 was substantially pure, the ATM-1 titer of the NBT-ATM-1 sample was assayed by EIA in the manner as described later in Example 5. The obtained ATM-1 titer (absorbance at 492 nm) of the sample was plotted against the amount of the sample. As a result, an approximately linear calibration line was obtained as shown in Fig. 8 (B). Therefore, this sample was proved to be substantially pure, showing that the sample can be suitably used as a standard ATM-1 sample in the immunoassay of ATM-1.

Run 2 (Confirmation of the removal of glycoproteins other than an ATM-1)

On the other hand, the 0.2 M α-methylmannose-eluted eluate obtained above was subjected to ultrafiltration in substantially the same manner as mentioned above to concentrate and dialyse it, thereby to obtain 5 ml of an α-methylmannose-free PB solution (0.2 M α-methylmannose-eluted eluate fraction). Separately, substantially the same procedures as mentioned above were repeated except that the 0.4 M α-methylmannose-eluted eluate obtained above was used in place of the 0.2 M α-methylmannose-eluted eluate, thereby to obtain 5 ml of a α-methylmannose-free PB solution (0.4 M α-methylmannose-eluted eluate fraction). To the above-mentioned purified ATM-1 sample was separately added each of 5 v/v% 0.2 M α-methylmannose-eluted eluate fraction and 5 v/v% 0.4 M α-methylmannose-eluted eluate fraction, and the ATM-1 titer of each of the resulting mixtures was assayed by the same method as mentioned above. The obtained titer (absorbance at 492 nm) of each mixture was plotted against the amount of the ATM-1 in the mixture. As a result, an approximately linear calibration line was not obtained as shown in Fig. 8 (B), but curves respectively formed by connecting the circles (O) and connecting the triangles (Δ) as shown in Fig.

8 (C) were obtained. In Fig. 8 (C), the curve formed by connecting the circles represents the results of the immunoassay of the mixture containing 0.2 M α-methylmannoseeluted eluate fraction and the curve formed by connecting the triangles represents the results of the immunoassay of the mixture containing 0.4 M α-methylmannose-eluted eluate fraction.

Further, substantially the same procedures as mentioned in Run 1 of Example 1 were repeated to culture the human tumor cell line NBT-2 and obtain the supernatant of the culture of the human tumor cell line. Then, from the supernatant, the ATM-1 was obtained through purification using a ConASepharose 4B beads in substantially the same manner as described in Run 1 of Example 1 except that in conducting the purification of the ATM-1 using ConASepharose beads, elutions using as eluents 0.2 M α-methylmannose-containing PB solution and 0.4 M α-methylmannose-containing PB solution were omitted and, instead, elution was conducted using 0.5 M α-methylmannose-containing PB solution only. Thus, there was obtained 5 ml of a sample solution containing the ATM-1. The ATM-1 titer of the thus obtained sample solution was assayed by the method as described in Run 1 of Example 1. The obtained titer was plotted against the amount of the ATM-1 in the sample solution. As a result, there was not obtained a substantially linear line as shown in Fig. 8 (B), but a curve as shown in Fig. 8 (A).

The above-mentioned results as shown in Figs. 8 (A) to (C) indicate that the supernatant contained, as impurities, glycoproteins other than the ATM-1 and such glycoproteins hindered the enzyme immunoassay (EIA) of the ATM-1.

Example 2

Preparation of an anti-ATM-1 monoclonal antibody:

Substantially the same procedures as described in Run 1 of Example 1 were repeated to obtain 1 ml of an ATM-1 solution containing the ATM-1 at a concentration of 20 mg-protein/ml.

1 ml of the ATM-1 solution was subjected to SDS-polyacrylamide gel electrophoresis (gel concentration: 10 w/v%) according to a method as described in Immunological Methods, 1979, published by Academic Press, N.Y. and a method as described in Immunological Method, volume II, 1981, published by Academic Press, N.Y. After electrophoresis, of the resulting gel, a gel portion corresponding to a substance having a molecular weight of about 120,000 ± 10,000 was cut out and broken into small pieces. To the small pieces of the gel was added 10-fold volume of a PB solution containing 10 w/v% sodium dodecyl sulfate (hereinafter referred to as "SDS"), followed by shaking at 4 °C overnight. The resulting mixture was subjected to centrifugation at 10,000 r.p.m. for 10 min. to separate it into a supernatant and the gel pieces. The supernatant was subjected to ultrafiltration using the same ultrafilter as mentioned above (Amicon PM-10) to increase the concentration. The concentrated supernatant was dialysed against PB, thereby to obtain 1 ml of a purified ATM-1 solution which had a protein concentration of 150 μg/ml.

1 ml of the above-obtained purified ATM-1 solution was mixed with 1 ml of a complete Freund's adjuvant (manufactured and sold by Difco Labora tories, U.S.A.) to obtain an emulsion. Then, 0.1 ml of the thus obtained emulsion was intraperitoneally administered to each of five BALB/C mice. Two weeks later, 0.1 ml of the above-obtained purified ATM-1 solution was administered to each of the five mice through their tail veins. Three days later, the spleens of the mice were excised from their bodies. The spleens were minced in a Hanks' solution (manufactured and sold by Nissui Pharmaceutical Co., Ltd., Japan) to obtain a spleen cell suspension. The spleen cell suspension was centrifuged at 800 r.p.m. for 10 min. to separate it into a supernatant and a cell pellet of the spleen cells. To the cell pellet was added 100 ml of an RPMI 1640 medium to prepare a spleen cell suspension having a cell concentration of $5 \times 10^5$ cells/ml. The spleen cell suspension was mixed with 100 ml of an RPMI medium in which mouse myeloma P3U1 cells (manufactured and sold by Immuno Biological Laboratories, Japan) were suspended at a cell concentration of $5 \times 10^5$ cells/ml. The resulting mixture was centrifuged at 800 r.p.m. for 10 min. to separate it into a supernatant and a cell pellet containing both the spleen cells and the mouse myeloma P3U1 cells. To the cell pellet was added 2 ml of an RPMI 1640 medium containing 50 w/v% of polyethylene glycol (PEG 4000, manufactured and sold by Merck & Co., Inc., West Germany), followed by incubation at 37 °C for 7 min. To the resulting mixture was gradually added 100 ml of an RPMI 1640 medium. Then, the mixture was centrifuged at 800 r.p.m. for 10 min. to separate it into a supernatant and a cell pellet. To the cell pellet was added 250 ml of an RPMI 1640 medium containing 20 v/v% FCS and the resulting mixture was stirred, thereby to obtain a uniform suspension. 10 μl of the thus obtained suspension was poured into each well of a 96 well-microplate (manufactured and sold by Coster, U.S.A.), each well of which has a capacity of 200 μl. Then, the suspension in each well was incubated overnight at 37 °C under the atmosphere of 5 v/v% of

$CO_2$. Further, to the suspension was added 10 µ1 of an HAT medium described in "Hybridoma Method and Monoclonal Antibodies", Takeshi Watanabe et al., published by R & D Planning Co., Ltd., Japan, 1982. The resulting suspension in each well was cultured at 37 °C under the atmosphere of 5 v/v% of $CO_2$. Two weeks later, hybridomas proliferatred in the suspensions in some wells. The same procedures as mentioned above were conducted using a number of microplates. Thus, there were obtained cultures of 1750 strains of hybridomas. From the thus obtained 1750 strains of hybridoma, hybridomas that produced, during the above-mentioned proliferation, a monoclonal antibody against the ATM-1 obtained in Example 1 was screened. The screening was conducted as follows.

Using a human tumor cell line NBT-2, a purified ATM-1 solution was prepared in substantially the same manner as described above. Then, 1 µl of the thus prepared purified ATM-1 solution was applied to each of the sections of a nitrocellulose filter which was given a lattice pattern to form sections (manufactured and sold by Toyo Filter Paper Co., Ltd., Japan), followed by air-drying. Then, the dried nitrocellulose filter was cut into pieces along the lattice pattern, and the pieces were separately put in each well of a 96 well-microplate, each well of which has a capacity of 200 µl. To the piece in each well was added 200 µ1 of a phosphate buffered saline solution containing 1 w/v% of bovine serum albumin (hereinafter often referred to as "BSA-PBS"), and the resulting mixture in each well was allowed to stand at room temperature for 2 hours, thereby to perform blocking of a plate. Then, the supernatant of each of the above-obtained cultures of the 1750 strains of hybridoma were separately added to each well of the 96 well-microplates in an amount of 100 µl, followed by incubation at room temperature for 1 hour. Then, each well was washed 3 times with PB. Then, to each well was added 100 µ1 of a biotinylated anti-mouse IgG of Vectastain ABC kit (manufactured and sold by Funakoshi Pharmaceutical Co., Ltd., Japan), and the resulting mixture in each well was allowed to stand at room temperature for 1 hour. Then, each well was washed 3 times with PB. Then, to each well was added 100 µ1 of an ABC reagent of the Vectastain ABC kit (manufactured and sold by Funakoshi Pharmaceutical Co., Ltd., Japan), and the resulting mixture in each well was allowed to stand at room temperature for 1 hour. Each well was washed 5 times with PB. Then, to each well was added a color-producing reagent for a peroxidase reaction (prepared by adding 2 ml of methanol containing 3 mg/ml 4-chloro-1-naphtol and 10 µl of 30 w/v% hydrogen peroxide to 10 ml of PB), followed by incubation at room temperature for 30 min., and occurrence of a color-development was examind. Of the mixtures in the wells, those mixtures which were dyed blue due to occurrence of the color development were regarded as positive, that is, the original supernatants used for such mixtures were regarded as positive. In this way, the hybridomas from which the supernatants regarded as positive were derived were selected. Thus, of the 1750 strains of hybridomas, 24 strains were selected. The thus obtained 24 strains produced anti-ATM-1 monoclonal antibodies. From these 24 strains producing anti-ATM-1 monoclonal antibodies, those hybridomas which were excellent in antibody-producing ability and in proliferating ability were selected. Thus, there were obtained 7 hybridomas and they were designated N2421, N7072, N9261, N1977, N3216, N1019 and N3233.

With respect to the monoclonal antibodies produced by those hybridomas, the abilities to bind to 4 kinds of the ATM-1, namely 4 glycoproteins as mentioned before, was examined. As a result, it was found that all the monoclonal antibodies bound to the glycoprotein having a molecular weight of 120,000. Further, the monoclonal antibodies produced by hybridomas N2421 and N7072 bound to all of the 4 glycoproteins. Hence, the hybridomas N2421 and N7072 are useful in determining the total amount of 4 kinds of the glycoproteins by EIA. On the other hand, the binding ability of monoclonal antibody produced by the hybridoma N3233 to higher molecular weight ATM-1, namely glycoproteins having molecular weights of about 1,100,000, about 900,000, and 700,000, respectively is extremely strong as compared with that of the monoclonal antibody to the lower molecular weight ATM-1, namely the glycoprotein having a molecular weight of about 120,000. Hence, if the antibody produced by the hybridoma N2421 or N7072 and the antibody produced by the hybridoma N3233 were respectively employed as a primary antibody and a secondary antibody for the immunoassay, the amount of only the higher molecular weight glycoproteins mentioned above can be selectively and effectively determined.

Example 3

Purification of an ATM-1 by affinity chromatography using an anti-ATM-1 monoclonal antibody:

50 mg of a monoclonal antibody produced by the hybridoma N7072 deposited at the European Collection of Animal Cell Cultures under an accession number 87012801 was bonded to 4 g of a CNBr-activated Sepharose 4B (manufactured and sold by Pharmacia Fine Chemicals AB, Sweden) in accordance with the description in the user's manual of the Sepharose 4B, thereby to obtain 10 ml of a monoclonal antibody-bonded Sepharose 4B beads. Then, the amount of the antibody remaining unreacted in the above-mentioned reaction was determined, and the amount of the reacted antibody was then determined by subtracting the amount of the antibody remaining unreacted from the original amount (i.e., 50 mg) of the antibody used for the reaction to give 35 mg. From the thus obtained amount of the reacted antibody, the amount of the antibody bonded to the Sepharose 4B beads was calculated. The amount of the antibody bonded to the Sepharose 4B beads was 3.5 mg per ml of the Sepharose B beads. Then, 10 ml of the NBT-ATM-1 as obtained in Example 1 was added to 1 ml of the monoclonal antibody-bonded Sepharose 4B beads, and then the mixture was shaken at room temperature for 5 hours. Then, the mixture was put in a minicolumn having a diameter of 10 mm and a length of 100 mm (manufactured and sold by BIO-RAD Co., Japan). The column was washed with 200 ml of PB to remove impurities. Then, to the minicolumn was added 10 ml of 0.2 M glycine hydrochloride (pH 2.0), thereby to elute out an ATM-1 from the antibody-bonded Sepharose 4B beads and to obtain an eluate containing the ATM-1. The eluate was subjected to ultrafiltration using Amicon PM-10 ultrafilter, and the eluate was concentrated and dialysed against PB in substantially the same manner as in Example 1, to obtain 1 ml of a purified ATM-1 solution. The protein concentration of the purified ATM-1 solution was determined based on an adsorption at 280 nm as in Example 1 and found to be 150 µg/ml. The purified ATM-1 solution was subjected to SDS-polyacrylamide gel electrophoresis according to the method as described in Immunological Methods, 1979, published by Academic Press, N.Y., U.S.A. or Immunological Method, volume II, 1981, published by Academic Press, N.Y., U.S.A. As a result, in the case where a gel containing 10 w/v% polyacrylamide was employed, a protein band was detected at a position corresponding to a molecular weight of about 120,000. On the other hand, in the case where a gel containing 3 w/v% polyacrylamide was employed, in addition to the above-mentioned band, a broad protein band was detected at a position corresponding to a molecular weight of about 500,000 to 1,000,000. Then, according to a generally known Western blot technique [H. Towbin et al; Proc. Natl, Acad, Sci, U.S.A. 764350 (1979)], the protein bands on each gel were transferred to a nitrocellulose membrane. Then, using a monoclonal antibody produced by the hybridoma N2421, to which a peroxidase was bonded, whether or not the monoclonal antibody bound to those protein bands was examined. As a result, it was found that the monoclonal antibody bound to all the protein bands. Therefore, it was proved that the ATM-1 comprised plural molecules of different molecular weights and that the ATM-1 obtained in Example 3 was substantially pure because no other protein bands were detected than those to which the anti-ATM-1 monoclonal antibody bound.

Example 4

Preparation of an immunoassay kit for an ATM-1:

(1) Production of an anti-ATM-1 monoclonal antibody:

$5 \times 10^5$ cells of the hybridoma N7072 was innoculated to 0.5 ml of an RPMI medium. The resulting mixture was intraperitoneally administered to a BALB/c mouse to which 0.5 µ1 of pristine (2,6,10,14,-tetramethyl-pentadecane, manufactured and sold by Sigma Chemical Company, U.S.A.) had intraperitonealy been administered 1 week before the administration of the mixture. 10 Days later, an accumulation of the ascites was observed in the abdominal cavity of the administered mouse. The ascites was taken out of the mouse using a syringe, followed by centrifugation at 1,000 r.p.m. for 10 min. to remove the hybridoma cells contained therein. Thus, there was obtained 3 ml of the ascites containing no hybridoma. To the thus obtained ascites were added 3 ml of a 20 mM Tris-hydrochloric acid buffer (pH 7.4) containing 20 mM sodium chloride (hereinafter often referred to as "TB"). To the resulting mixture was added ammonium sulfate while cooling on ice so that the saturation degree of the ammonium sulfate in the resulting mixture became 50 %. The resulting mixture was allowed to stand for 1 hour while cooling on ice, followed by centrifugation at 10,000 r.p.m. for 10 min. to separate the mixture into a supernatant and a pellet. The pellet was dissolved in TB and the resulting solution was dialysed against TB to remove the ammonium sulfate. Then, the dialysed solution was applied to a DEAE-cellulose column (manufactured and sold by Pharmacia Fine Chemicals AB, Sweden) having a gel volume of 20 ml which had previously been equilibrated with TB. Then, 500 ml of TB was flowed through the column to wash away protein impurities. Further, 100 ml of a Tris-hydrochloric acid buffer (pH 7.4) containing 100 mM NaCl was flowed through the column to liberate an antibody from the DEAE-cellulose, thereby to obtain an antibody fraction. Then, the antibody fraction was subjected to ultrafiltration using a ultra-filter (Amicon PM-10, manufactured and sold by Amicon Co., Ltd., U.S.A.) to concentrate the fraction. The concentrated fraction was dialysed against PB to obtain a PB solution containing of a monoclonal antibody produced by the hybridoma N7072. The amount of the monoclonal antibody thus obtained was 9 mg per mouse.

On the other hand, substantially the same procedures as described above were repeated except that a monoclonal antibody produced by the hybridoma N2421 was employed in place of the monoclonal antibody produced by the hybridoma N7072. As a result, a PB solution was obtained containing a monoclonal antibody produced by the hybridoma N2421. The amount of the monoclonal antibody thus obtained was 11 mg per mouse.

(2) Preparation of an assay plate:

The above-obtained PB solution containing the monoclonal antibody produced by the hybridoma N7072 was diluted with PB so that the concentration of the antibody in the resulting solution became 50 µg/ml. 10 µl of the resulting solution was put in each well of an Immulon 600 plate having 96 wells of a capacity of 200 µl (manufactured and sold by C.A.Greiner und Sohne, Nurtingen, West Germany), followed by incubation at 4 °C for 2 days, thereby to immobilize the antibody on the bottom of each well. The immobilized antibody was washed with PB once, and then 200 µl of a PBS containing 1 w/v% bovine serum albumin (hereinafter often referred to as BSA-PBS) was put in each well of the plate, followed by incubation at 4 °C to perform blocking of the plate.

(3) Preparation of a secondary antibody to which a peroxidase was bonded:

To the monoclonal antibody produced by the hybridoma N2421, a peroxidase was bonded as a labeling agent according to a customary known method [E. Ishikawa et al: J. Immunoassay 4, 209 (1983)] as follows.

The PB solution containing the monoclonal antibody produced by the hybridoma N2421 as obtained in Step (1) above was dialysed against 0.1 M phosphate buffer (pH 6.5), thereby to obtain a monoclonal antibody solution having a monoclonal antibody concentration of 10 mg/ml. To 1 ml of the thus obtained monoclonal antibody solution was added 20 µl of a solution prepared by dissolving 9.1 mg of S-acetylmercaptosuccinic anhydride in 150 µ1 of N,N-dimethyl formamide, and then the resulting mixture was stirred with a magnetic stirrer at room temperature for 30 min. to advance a reaction. Further, to the reaction mixture were added 200 µ1 of 0.1 M Tris-hydrochloric acid buffer (pH 7.0), 40 µl of 0.1 M ethylenediaminetetraacetic acid (pH 7.0) and 100 µ1 of 1 M hydroxylamine solution (pH 7.0), and then the resulting mixture was stirred at 30 °C for 4 min. to advance a reaction. The reaction mixture was subjected to ultrafiltration using the same ultrafilter as used in Step (1) above to concentrate the reaction mixture. The concentrated solution was dialysed against 0.1 M phosphate buffer (pH 6.0) containing 5 mM ethylenediaminetetraacetic acid, thereby to obtain 1 ml of an antibody solution containing a monoclonal antibody produced by the hybridoma N2421, in which thiol groups were introduced.

On the other hand, to a solution obtained by dissolving 20 mg of a peroxidase (manufactured and sold by Sigma Chemical Company, U.S.A.) in 3 ml of 0.1 M phosphate buffer (pH 7.0) was added a solution obtained by dissolving 16 mg of N-succinimidyl 4-(N-maleimidomethyl) cyclohexane-1-carboxylate in 200 µ1 of N,N-dimethylformamide. The resulting mixture was stirred at 30 °C for 2 hours to advance a reaction. In the reaction mixture, precipitates were formed.

The mixture was centrifuged at 3000 r.p.m. for 10 min. to separate the mixture into a supernatant and the precipitates. The supernatant was subjected to ultrafiltration using the same ultrafilter as used above to concentrate the supernatant. The concentrated supernatant was dialysed against 0.1 M phosphate buffer (pH 6.0), thereby to obtain 1 ml of a peroxidase solution containing a peroxidase in which thiol groups were introduced. The thus obtained peroxidase solution was mixed with the above-obtained antibody solution, and then the mixture was allowed to stand at 30 °C for 1 hour to advance to reaction. The reaction mixture was applied to a column packed with Sepharose 6B (manufactured and sold by Pharmacia Fine Chemicals AB, Sweden). Then, the elution was conducted using as an eluent 0.1 M phosphate buffer, and a fraction corresponding to a molecular weight of about 200,000 was collected. Thus, there was obtained 15 ml of a solution containing 200 µg/ml peroxidase-bonded monoclonal antibody derived from the hybridoma N2421.

Example 5

Immunoassay for determining the presence of the ATM-1:

The assay plate prepared in Example 4 was washed with PB three times. On the other hand, the ATM-1 obtained in Example 1 from the supernatant of a culture of a human tumor cell line NBT-2 was diluted 10-fold with a PB solution containing 0.5 % w/v bovine serum albumin (BSA) (hereinafter often referred to as "BSA-PB") to obtain a standard ATM-1 solution containing the ATM-1 in an amount of 100 U/ml. The standard ATM-1 solution was diluted with BSA-PB by doubling the dilution. The resulting diluted solutions each were separately poured into the wells of the above-mentioned assay plate in an amount of 60 µl per well. The assay plate was allowed to stand at room temperature for 15 hours to bind the ATM-1 in the diluted solution to the primary antibody immobilized on the bottom of each well of the assay plate. The plate was washed with PB 5 times. On the other hand, the solution of a peroxidase-bonded monoclonal antibody obtained in Example 4 was diluted 10-fold with 0.1 M phosphate buffer (pH 7.0) containing 0.2 w/v% BSA and 1 v/v% mouse serum to obtain a secondary antibody solution. 80 µl of the secondary antibody solution was added to each well of the assay plate, and the assay plate was allowed to stand at room temperature for 2 hours and washed with PB seven times. Then, to each well of the assay plate was added 100 µ1 of a peroxidase substrate solution so that a reaction between the peroxidase and substrate proceeded. The peroxidase substrate solution was prepared by mixing a solution obtained by dissolving 50 mg of o-phenylenediamine in 10 ml of 0.1 M citrate-$Na_2HPO_4$ buffer (pH 5.0) and a solution obtained by adding 10 µl of 30 % $H_2O_2$ to 10 ml of distilled water. 30 Minutes later, 100 µl of 1 N HCl was added to each well of the assay plate to terminate the reaction. Then, the absorbance of each of the reaction mixture was measured at 492 nm ($OD_{492}$) using Titertek Multiskan (a spectrphotometer manufactured and sold by Flow Laboratories, Co., Ltd., U.S.A.). The above-mentioned procedures were repeated twice and the three absorbance values obtained were averaged. As a control, substantially the same procedures as mentioned above were repeated except that BSA-PB was used instead of the standard ATM-1 solution.

The results are shown in Fig. 1. As is apparent from Fig. 1, there is a substantially linear relationship between the ATM-1 concentration and $OD_{492}$. This indicates that by the immunoassay as described in Example 5, the ATM-1 concentration in a specimen can be determined accurately.

Example 6

Molecular weight of the ATM-1 obtained from the supernatant of a culture of a human tumor cell line NBT-2:

The molecular weight of the ATM-1 obtained in Example 1 from the supernatant of a culture of a human tumor cell line NBT-2 derived from a human urinary bladder cancer was determined by the method as described before. The result is shown in Fig. 2. As is apparent from Fig. 2, the ATM-1 obtained from the supernatant of a culture of a human tumor cell line NBT-2 comprises 3 antigens having molecular weights of about 1,100,000, about 900,000 and about 120,000, respectively.

Example 7

Molecular weight of the ATM-1 present in the serum of a patient suffering from a cancer:

Using as a sample 0.5 ml of the serum of a patient suffering from a lung cancer, the molecular weight of the ATM-1 in the serum was determined by the method as described before. The result is shown in Fig. 3. As is apparent from Fig. 3, the ATM-1 in the serum of a patient suffering from a lung cancer comprises 4 antigens having molecular weights of about 1,100,000, about 900,000, about 700,000 and about 120,000, respectively.

On the other hand, using as a sample 0.5 ml of the serum of a patient suffering from a breast carcinoma, the molecular weight of the ATM-1 in the serum was determined by the method as described before. The result is shown in Fig. 4. As is apparent from Fig. 4, the ATM-1 in the serum of a patient suffering from a breast carcinoma comprises 2 antigens having molecular weights of about 900,000 and about 120,000, respectively.

Example 8

Isoelectric point of the ATM-1 having a molecular weight of about 900,000 obtained from the serum of a patient suffering from a breast carcinoma:

The fractions containing the ATM-1 of a molecular weight of about 900,000 (fraction Nos. 26 to 30 obtained in Example 7) were pooled and condensed using Molcut SJTK01324 (an ultrafiltration unit manufactured and sold by Nihon Millipore Ltd., Japan) to obtain a 0.5 ml sample. The sample was subjected to chromatofocusing by the method as described hereinbefore. The result is shown in Fig. 5. As is apparent from Fig. 5, the ATM-1 of a molecular weight of about 900,000 exhibited an isoelectric point of about 4.2.

Example 9

Isoelectric point of the ATM-1 present in the serum of a patient suffering from a cancer and the supernatant of a culture of a human tumor cell line:

Substantially the same procedures as described in Example 8 were repeated except that there was used the ATM-1 which was obtained from the serum of a patient suffering from a lung cancer or the supernatant of a culture of a human cell line NBT-2 and had a molecular weight as described in Table 1 below, thereby to determine the isoelectric point of the ATM-1. The results are shown in Table 1 together with the isoelectric point of the ATM-1 having a molecular weight of about 900,000 which was obtained from the serum of a patient suffering from a breast carcinoma.

Table 1

| Sample | Molecular weight | Isoelectric point |
|---|---|---|
| Serum of a patient suffering from a breast carcinoma | about 900,000 | about 4.2 |
| Serum of a patient suffering from a lung carcinoma | about 1,100,000<br>about 700,000<br>about 120,000 | about 4.0<br>about 4.8<br>about 6.5 |
| Supernatant of a culture of a human tumor cell line NBT-2 | about 1,100,000<br>about 900,000 | about 4.0<br>about 4.2 |

Example 10

Binding ability of the ATM-1 to a lectin:

An aliquot of the ATM-1-containing PB solution obtained in Example 1 was diluted 10-fold with BSA-PB. Then, the diluted solution was further diluted with BSA-PB by doubling the dilution to obtain a series of the diluted solutions. Substantially the same procedures as in Example 5 were repeated except that the series of the diluted solutions were used as a sample and that lectin solutions containing 1 µg/ml peroxidase-bonded succinyl ConA (manufactured and sold by Vector Co., U.S.A.), 1 µg/ml peroxidase-bonded PNA (manufactured and sold by Honen Co., Ltd., Japan) and 0.25 µg/ml peroxidase-bonded WGA (manufactured and sold by Honen Co., Ltd., Japan) were separately used instead of the secondary antibody solution, to advance the reactions between the ATM-1 and a lectin. The absorbance of the reaction mixture at 492 nm was measured. The above-mentioned procedures were conducted three times. The three absorbance values obtained were averaged. The results are shown in Fig. 6. As is apparent from the results, there is a substantially linear relationship between the amount of the ATM-1 and the absorbance.

On the other hand, substantially the same procedures as mentioned above were repeated except that two types of ATM-1 having molecular weights of about 1,100,000 and about 900,000, respectively were used as a sample instead of the ATM-1 obtained from the supernatant of a culture of a human tumor cell line NBT-2. As a result, it was found that these two types of ATM-1 obtained from the serum of a patient suffering from a lung cancer were capable of binding to Con A and WGA.

Example 11

The amount of ATM-1 in the supernatants of cultures of various human tumor cell lines:

Each of 11 human tumor cell lines, i.e., PC-1, PC-3, PC-6, PC-7, PC-9, PC-13, PC-14 and QG-56 derived from a lung cancer, MKN-45 and KATO-III derived from a gastric cancer and NBT-2 derived from a cancer of the urinary bladder was cultured in an RPMI 1640 medium containing 10 v/v% FCS for 5 days. The resulting culture was subjected to centrifugation as in Example 1 to obtain 100 ml of a supernatant. To the supernatant was added 1 ml of ConA-Sepharose (manufactured and sold by Pharmacia Fine Chemicals AB, Sweden) and the mixture was shaken at room temperature for 5 hours. The resulting mixture was subjected to elution as in Example 1 to obtain 1 ml of 0.5 M α-methylmannose-eluted eluate. The eluate was diluted 10-fold with BSA-PB and subjected to immunoassay as in Example 5, thereby to determine the amount of the ATM-1 in the eluate. The above-mentioned procedures were conducted three times and the amounts of the ATM-1 obtained were averaged. The results are shown in Table 2.

Table 2

| Human tumor cell lines | Absorbance at 492 nm | Amount of ATM-1 (U/ml) |
|---|---|---|
| PC-1 | 0.51 | 50 |
| PC-3 | 0.05 | 4 |
| PC-6 | 0.01 | < 1 |
| PC-7 | 0.02 | < 2 |
| PC-9 | 0.01 | < 1 |
| PC-13 | 0.37 | 38 |
| PC-14 | 0.90 | 89 |
| QG-56 | 0.15 | 14 |
| MKN-45 | 0.10 | 9 |
| KATO-III | 0.01 | < 1 |
| NBT-2 | 0.25 | 24 |

As is apparent from the results, the amount of the ATM-1 which can be obtained from the supernatant of a culture of a human tumor cell line was varied according to the kind of the human tumor cell line. The human tumor cell lines PC-1, PC-13, PC-14 and NBT-2 produced the ATM-1 in relatively high amounts, whereas the human tumor cell lines PC-6, PC-9 and KATO-III scarcely produce the ATM-1.

Example 12

The amount of ATM-1 in the serum of a patient suffering from a cancer and in normal human serum:

Using as specimens the sera of patients suffering of one of various cancers, the sera of patients suffering from a benign hepatic disease and normal human sera, the amount of the ATM-1 in the sera was determined as follows.

Each serum was diluted 6 times with 0.2 M phosphate buffer (pH 7.0), and subjecting to immunoassay as in Example 5 to determine the amount of the ATM-1 in each serum. The standard ATM-1 solutions for preparing a standard calibration curve to be used for the immunoassay were prepared as follows. The 1000 U/ml of the ATM-1 solution obtained in Example 1 from the supernatant of a culture of a human tumor cell line NBT-2 was added to a solution obtained by diluting 6-fold the normal human serum (the $OD_{492}$ of the solution is 0) so that solutions containing the ATM-1 at concentrations of 100, 50, 25 and 12.5 U/ml, respectively were prepared. The thus obtained solutions were subjected to the immunoassay as in Example 5 to obtain an ATM-1 standard calibration curve. The absorbance measured with respect to each sample was compared with the ATM-1 standard calibration curve to determine the amount of the ATM-1 in each sample. The above-mentioned procedures were conducted three times and the amounts of the ATM-1 in each sample obtained were averaged.

The results are shown in Fig. 7. As is apparent from Fig. 7, with respect to normal human serum, there was no sample which contained the ATM-1 at a concentration of 5 U/ml or more. If the sample containing the ATM-1 at a concentration of 5 U/ml or more is regarded as ATM-1 positive, 10 of 13 samples were ATM-1 positive in the case of a breast carcinoma, 11 of 16 samples in the case of a gastric cancer, 8 of 14 samples in the case of a lung cancer, and 13 of 14 samples in the case of a hepatoma. That is, the ATM-1 positive ratio (the ratio of the ATM-1 positive samples to the total samples subjected to immunoassay to determine the presence of the ATM-1) was 77 % in the case of a breast carcinoma, 69 % in the case of a gastric cancer, 57 % in the case of a lung cancer and 93 % in the case of a hepatoma.

As the serum of a benign hepatic disease, the sera of patients suffering from cirrhosis or chronic hepatitis were used and the amounts of the ATM-1 in the sera were determined. As a result, only one of 18 samples was found to be ATM-1 positive (the ATM-1 positive ratio was 5 %).

Incidentally, with respect to the patients suffering from a breast carcinoma whose sera were used in this Example, all the patients suffered from primary stage (stage I) of a breast carcinoma. With respect to the patients suffering from a gastric cancer whose sera were used in this Example, half of the patients suffered from primary stage of a gastric cancer and the remaining half of the patients suffered from progressive stage of a gastric cancer. Further, with respect to the patients suffering from a hepatoma and a lung cancer, all the patients suffered from progressive stage of the cancer.

## Claims

1. An antibody which is capable of binding to at least one antigen selected from the group consisting of:

(A) a glycoprotein having a molecular weight of about 1,100,000 ± 100,000 and having an isoelectric point of about 4.0 ± 0.2,

(B) a glycoprotein having a molecular weight of about 900,000 ± 100,000 and having an isoelectric point of about 4.2 ± 0.2,

(C) a glycoprotein having a molecular weight of about 700,000 ± 100,000 and having an isoelectric point of about 4.8 ± 0.2, and

(D) a glycoprotein having a molecular weight of about 120,000 ± 20,000 and having an isoelectric point of about 6.5 ± 0.5;

and wherein at least one of said antigen is present in the serum of a patient suffering from a hepatoma, a breast carcinoma, a gastric cancer or a lung cancer, and wherein at least one of said antigen is capable of binding to at least one monoclonal antibody selected from the group consisting of:

a monoclonal antibody produced by the hybridoma N2421 deposited at the European Collection of Animal Cell Cultures under the accession number 87050805;

a monoclonal antibody produced by the hybridoma N7072 deposited at the European Collection of Animal Cell Cultures under the accession number 87012801; and

a monoclonal antibody produced by the hybridoma N9261 deposited at the European Collection of Animal Cell Cultures under the accession number 87012802.

2. An antibody according to claim 1, which is a monoclonal antibody.

3. A monoclonal antibody which is produced by the hybridoma N2421 deposited at the European Collection of Animal Cell Cultures under the accession number 87050805.

4. A monoclonal antibody which is produced by the hybridoma N7072 deposited at the European Collection of Animal Cell Cultures under the accession number 87012801.

5. A monoclonal antibody which is produced by the hybridoma N9261 deposited at the European Collection of Animal Cell Cultures under the accession number 87012802.

6. An antigen which is a glycoprotein present in the serum of a patient suffering from a hepatoma, a breast carcinoma, a gastric cancer or a lung cancer, and which is capable of binding to at least one monoclonal antibody selected from the group consisting of:

a monoclonal antibody produced by the hybridoma N2421 deposited at the European Collection of Animal Cell Cultures under the accession number 87050805;

a monoclonal antibody produced by the hybridoma N7072 deposited at the European Collection of Animal Cell Cultures under the accession number 87012801; and

a monoclonal antibody produced by the hybridoma N9261 deposited at the European Collection of Animal Cell Cultures under the accession number 87012802.

7. An antigen according to claim 6, which has a molecular weight of about 1,100,000 ± 100,000 and having an isoelectric point of about 4.0 ± 0.2.

8. An antigen according to claim 6, which has a molecular weight of about 900,000 ± 100,000 and having an isoelectric point of about 4.2 ± 0.2.

9. An antigen according to claim 6, which has a molecular weight of about 700,000 ± 100,000 and having an isoelectric point of about 4.8 ± 0.2.

10. An antigen according to claim 6, which has a molec ular weight of about 120,000 ± 20,000 and having an isoelectric point of about 6.5 ± 0.5.

11. The hybridoma N2421 which is deposited at the European Collection of Animal Cell Cultures under an accession number 87050805.

12. The hybridoma N7072 which is deposited at the European Collection of Animal Cell Cultures under an accession number 87012801.

13. The hybridoma N9261 which is deposited at the European Collection of Animal Cell Cultures under an accession number 87012802.

14. A method for detecting an antigen of claim 6 in a specimen derived from a human body, which comprises subjecting said specimen to immunoassay using an antibody of any one of claims 1 to 5.

15. A method for producing an antigen of claim 6, which comprises determining the presence of an antigen of claim 6 in a serum of a patient suffering from a cancer or a supernatant of a culture of a human tumor cell line and subjecting said serum or said supernatant to purification using a lectin.

16. A method according to claim 15, wherein said purification comprises:

(1) contacting said serum or said supernatant to a lectin to adsorb glycoproteins including said antigen on said lectin,

(2) eluting out glycoproteins other than said antigen from the resulting lectin using a 0.4 M saccharide solution as an eluent, and

(3) eluting out said antigen from the resulting lectin using as an eluent a saccharide solution having a saccharide concentration of more than 0.4 M.

17. A method according to any one of claim 15 and 16, wherein said cancer is selected from the group consisting of a hepatoma, a breast carcinoma a gastric cancer and a lung cancer.

FIG. 1

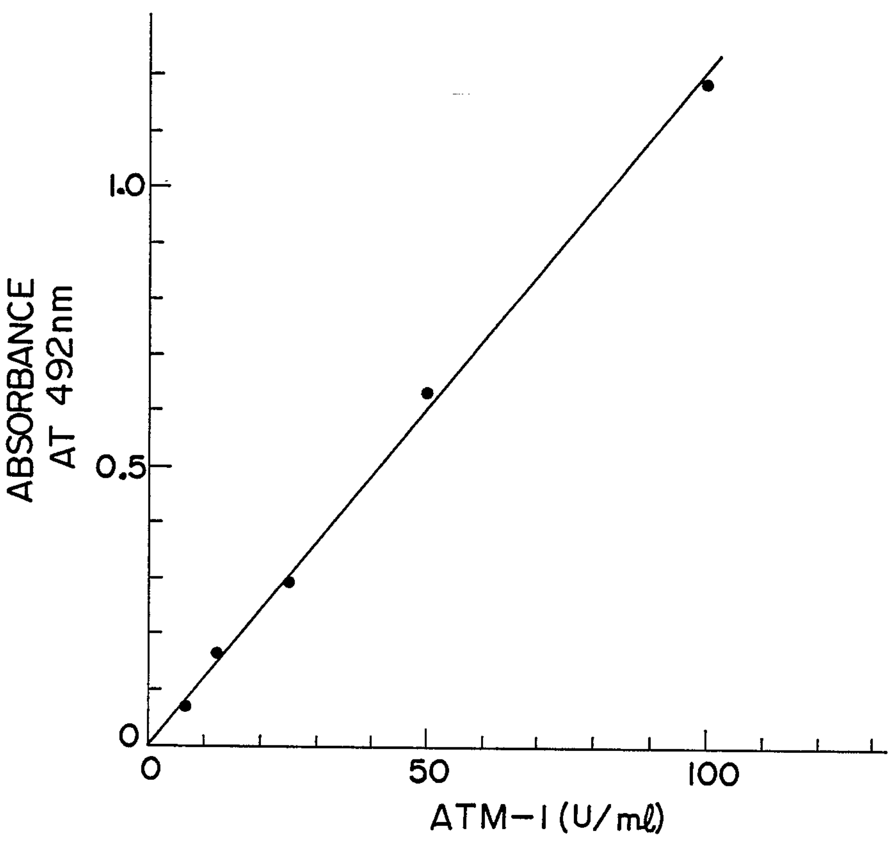

FIG. 2

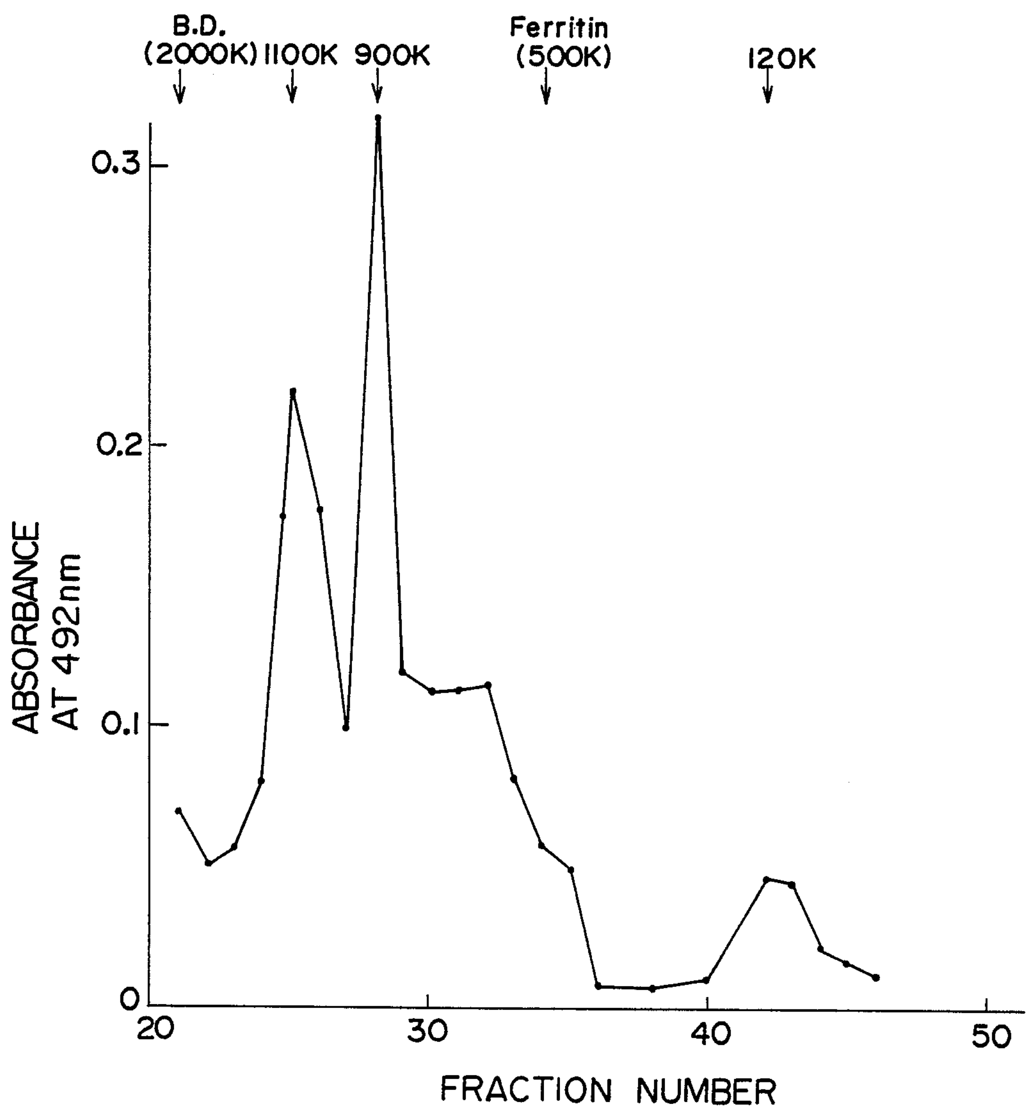
B.D.
(2000K)
1100K
900K
Ferritin
(500K)
120K
0.3
0.2
0.1
0
ABSORBANCE AT 492nm
20
30
40
50
FRACTION NUMBER

FIG. 3

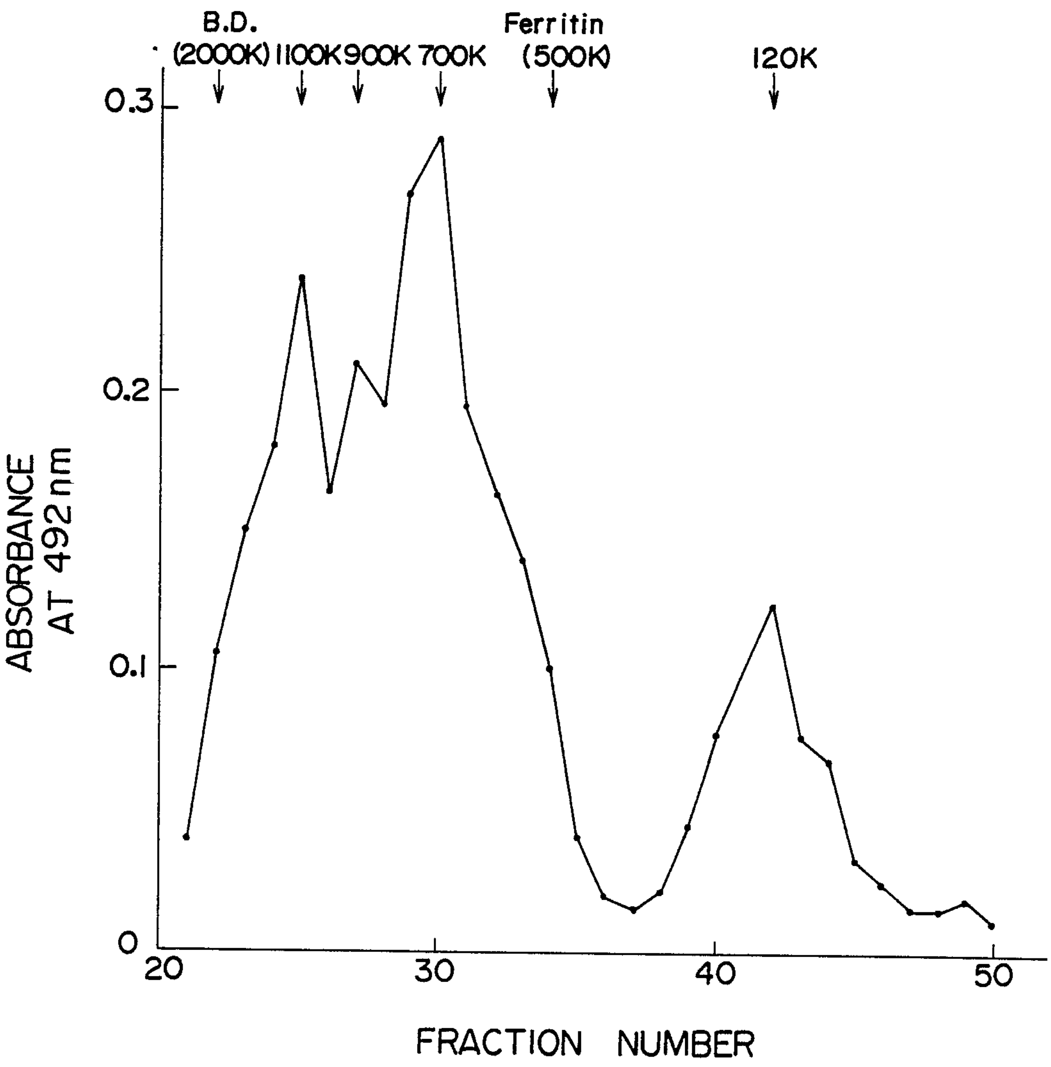
B.D.
(2000K)
1100K
900K
700K
Ferritin
(500K)
120K
0.3
0.2
0.1
0
ABSORBANCE AT 492 nm
20
30
40
50
FRACTION NUMBER

FIG. 4

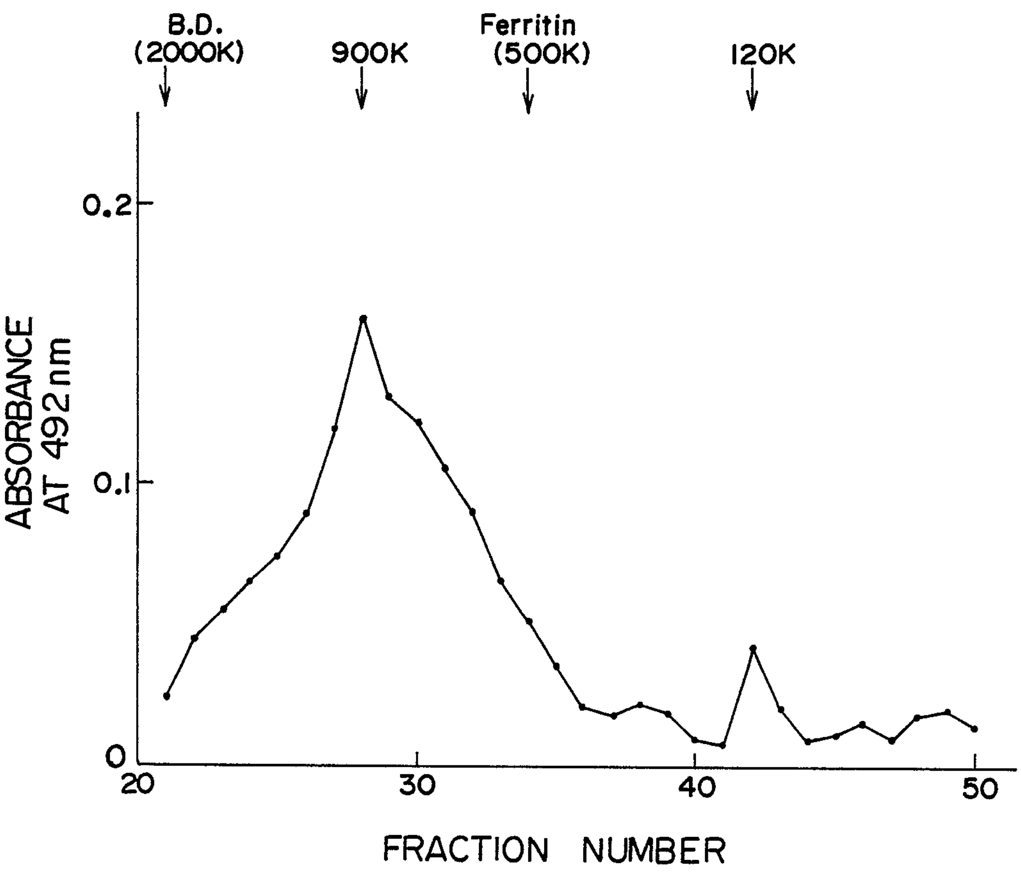
B.D.
(2000K)
900K
Ferritin
(500K)
120K
ABSORBANCE
AT 492 nm
0.2
0.1
0
20
30
40
50
FRACTION NUMBER

FIG. 5

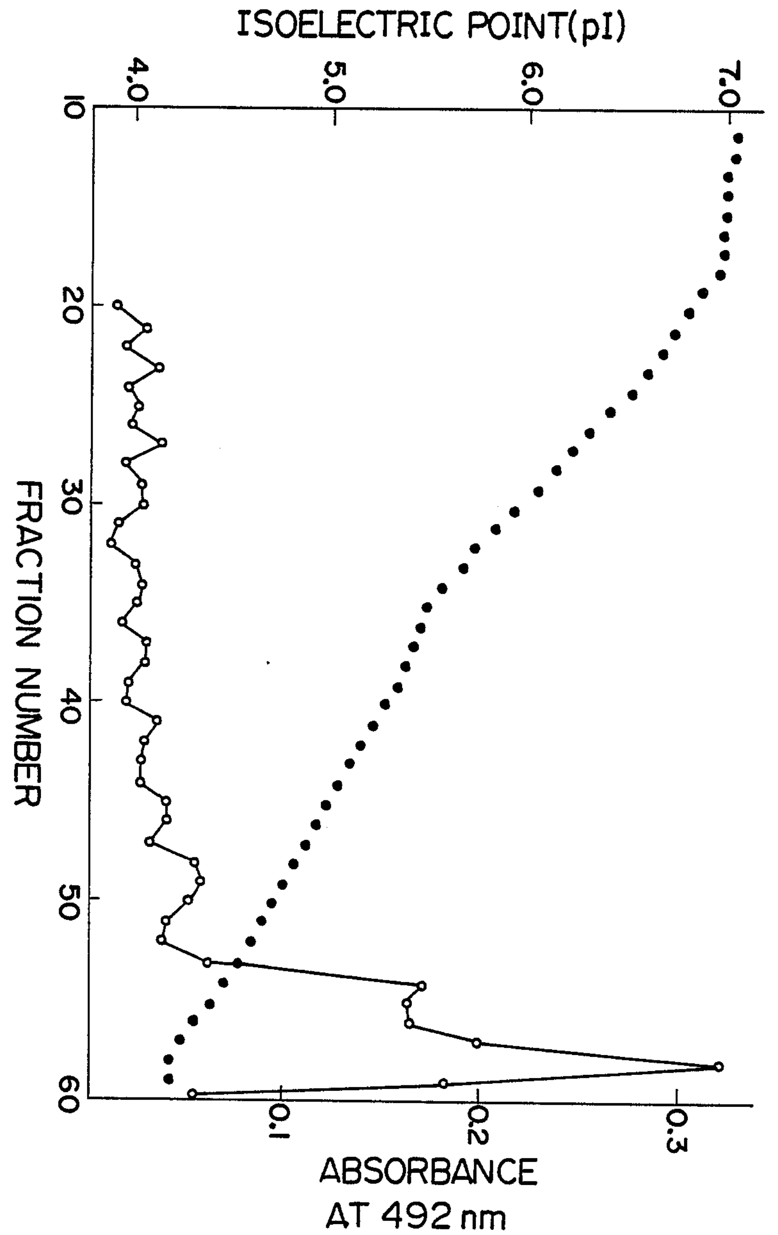
ISOELECTRIC POINT(pI)
4.0
5.0
6.0
7.0
10
20
30
40
50
60
FRACTION NUMBER
0.1
0.2
0.3
ABSORBANCE
AT 492 nm

FIG. 6 (A)

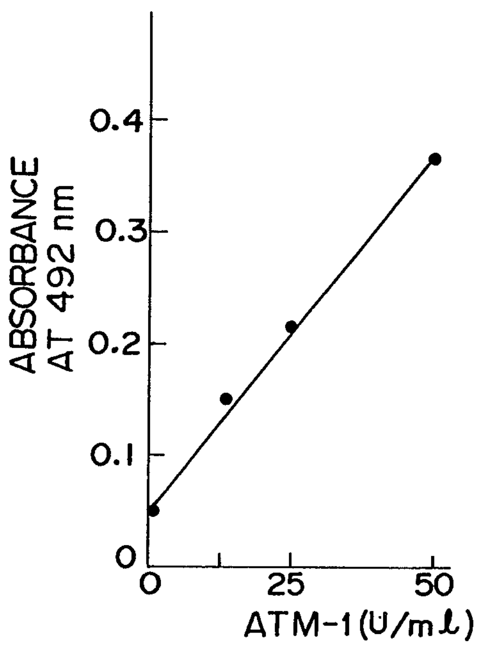


FIG. 6 (B)

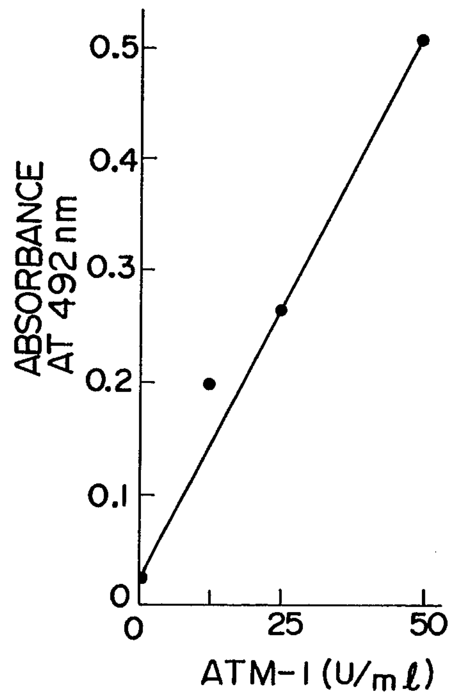


FIG. 6 (C)

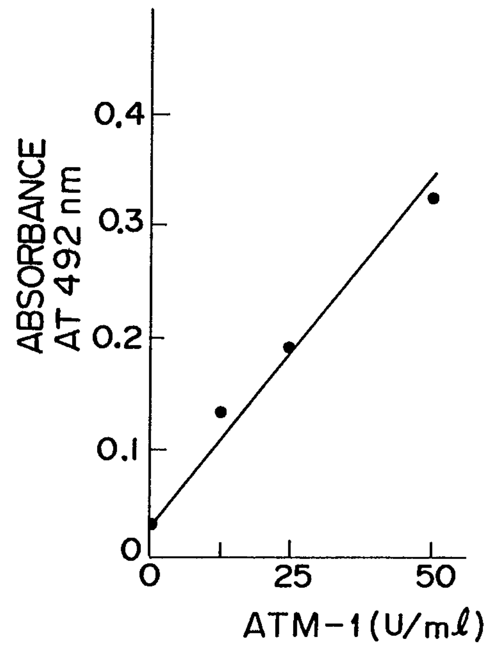

FIG. 7

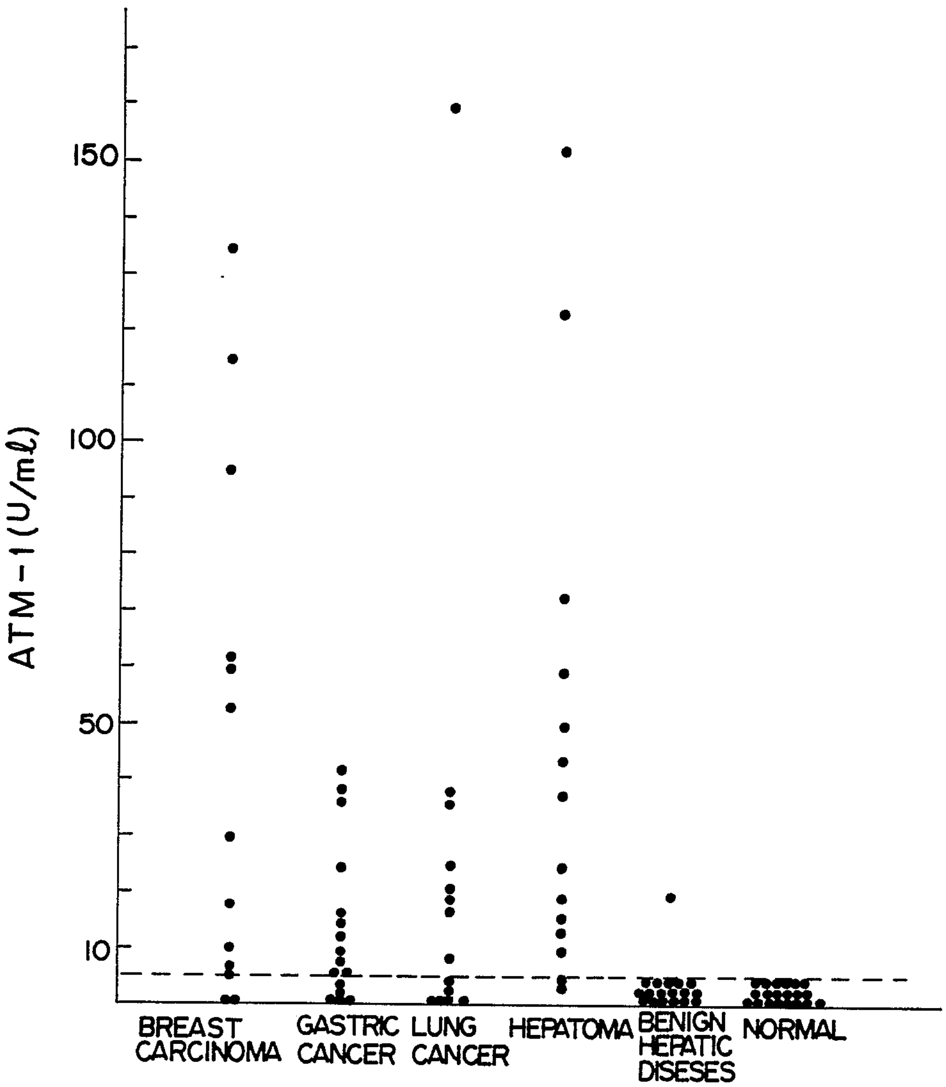
ATM-1 (U/mℓ)
150
100
50
10
BREAST CARCINOMA
GASTRIC CANCER
LUNG CANCER
HEPATOMA
BENIGN HEPATIC DISESES
NORMAL

FIG. 8 (A)

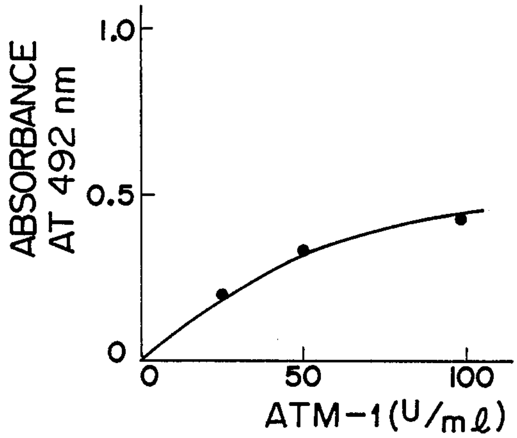


FIG. 8 (B)

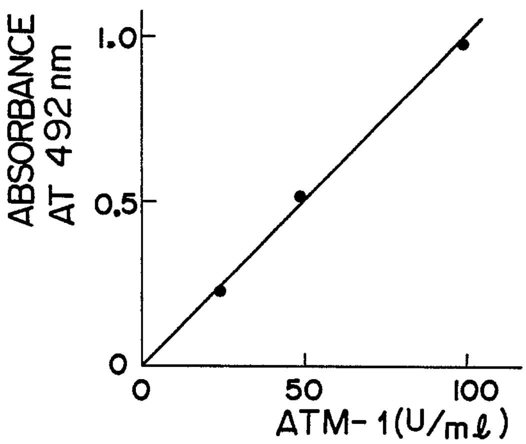


FIG. 8 (C)